# EUROPÄISCHE PATENTSCHRIFT

(11) **EP 2 181 188 B1**
(45) Veröffentlichungstag und Bekanntmachung des Hinweises auf die Patenterteilung: **12.06.2013**
(21) Anmeldenummer: 09728337.8
(22) Anmeldetag: 31.03.2009
(51) Int. Cl.: B01L 3/00, C12M 1/32

(54) **MIKROBIOREAKTOR SOWIE MIKROTITER-PLATTE MIT MEHREREN MIKROBIOREAKTOREN**
MICROBIOREACTOR AND MICROTITER PLATE COMPRISING A PLURALITY OF MICROBIOREACTORS
MICROBIORÉACTEUR ET PLAQUE DE MICROTITRATION COMPRENANT UNE PLURALITÉ DE MICROBIORÉACTEURS

(30) Priorität: 03.04.2008 DE 102008017765
(43) Veröffentlichungstag der Anmeldung: 05.05.2010
(73) Patentinhaber: Technische Universität Ilmenau, 98693 Ilmenau (DE)
(72) Erfinder: SCHOBER, Andreas, 90762 Fürth (DE); AUGSPURGER, Caroline, 76229 Karlsruhe (DE); WEISE, Frank, 98693 Ilmenau (DE); FERNEKORN, Uta, 99094 Erfurt (DE); HILDMANN, Christian, 99085 Erfurt (DE); HAMPL, Jörg, 99087 Erfurt (DE)
(74) Vertreter: Engel, Christoph Klaus
(86) Internationale Anmeldenummer: PCT/EP2009/053789
(87) Internationale Veröffentlichungsnummer: WO 2009/121868

(56) Entgegenhaltungen:
- WO-A2-03/085379
- DE-A1- 10 326 747
- US-A1- 2005 260 745
- US-A1- 2007 128 715

## Beschreibung

Die Erfindung betrifft im Allgemeinen kostengünstig herzustellende, vorzugsweise parallel anzuordnende mikrofluidische Systeme zur Kultivierung von fortgeschrittener Zellkultur (3D Kultur, Stammzellen etc.), die in jedem biotechnologisch und biomedizinisch arbeitenden Labor sinnvoll eingesetzt werden können und zudem HTS-fähig (HTS = High-Throughput-Screening) sind. Solche Systeme sind prinzipiell geeignet, in der Wirkstoffsuche bei ADME/Tox Screenings eingesetzt zu werden und weisen daher ein hohes Vermarktungspotential auf. ADME/Tox Studien dienen der Untersuchung von Substanzen auf ihre Eigenschaften im (menschlichen) Organismus in Bezug auf Absorption, Distribution, Metabolisierung, Exkretion und Toxizität. Im Speziellen betrifft die Erfindung einen Mikrobioreaktor mit einem Probenträger, der z.B. eine Zellkultur trägt und von einem Fluid durchströmt wird. Von einem Mikrobioreaktor im Sinne der Erfindung spricht man, wenn dieser (im statischen Zustand) eine Medienmenge im unteren Milliliter- vorzugsweise im Mikroliter-Bereich enthält. Außerdem bezieht sich die Erfindung auf eine CellChip Anordnung bzw. Mikrotiter-Platte, in welcher mehrere Mikrobioreaktoren integriert sind.

Für vielfältige Anwendungen in der Medizin und Pharmaforschung oder in der Kosmetikindustrie ist die Kultivierung biologischen Materials eine herausragende Aufgabe. Hierbei ist Zellmaterial, das können Gewebeschnitte z.B. aus Biopsien oder primäre Zellen, die aus Tieren oder Menschen gewonnen wurden, Zelllinien oder gentechnisch veränderte Zellen sein, möglichst so zu kultivieren, dass seine natürliche Funktion und Lebensfähigkeit beibehalten werden bzw. die angestrebte Funktion möglichst optimal umgesetzt wird.

Die Bandbreite der Anwendung reicht dabei vom Bereitstellen von Testsystemen für die Pharma- und Kosmetikforschung über die medizinische Forschung (z.B. die Stammzellforschung) bis zur Produktion von Impfstoffen. Ein wichtiger Aspekt ist dabei sowohl der sparsame Umgang mit wertvollem Zellmaterial als auch die Bereitstellung vieler verschiedener paralleler Ansätze zum Durchtesten von unterschiedlichen Parametern.

In einem Gewebe müssen Nährstoffe und Substanzen zur Differenzierung (Botenstoffe) nicht nur über Diffusion, sondern auch mit Hilfe von aktiven Transportmechanismen analog zum Blutkreislauf (und auch dem Lymphsystem) interzellulär zu- und abgeführt werden. Es hat sich gezeigt, dass hierbei die Geometrie z.B. von Unterstützungssystemen, Gerüsten zur Ansiedelung von Zellen, der Abstand und die Führung von fluidischen Versorgungskanälen sowie die Dichte, die Art und der Typus der Zellbesiedelung wichtige Parameter sind, die über die erfolgreiche Kultivierung bzw. Herstellung von Gewebe im Tissue Engineering entscheiden. Zudem ist die Induktion von Differenzierungsprozessen durch gezielte Wirkstoffabgabe in einem Gewebe ein ungelöstes Problem, das bislang nur über "trial and error" Methoden getestet wird.

Für die ordnungsgemäße Funktion eines Gewebes oder Organs sind viele Parameter von Bedeutung. Um die Aufrechterhaltung von Organ- und Gewebefunktionen *in vitro* zu gewährleisten, muss nicht nur die korrekte molekulare, sondern vor allem auch die korrekte makroskopische Architektur des Zellverbandes reproduziert werden. So ist bekannt, dass primäre Zellen häufig ihre zelltypspezifischen (differenzierten) Funktionen verlieren, wenn sie als Monolayer kultiviert werden. Es sind deshalb verschiedene Anstrengungen unternommen worden, Zellkultursysteme zu entwickeln, die die dreidimensionale *in vivo* Situation des korrespondierenden Gewebes besser nachbilden.

Eine Möglichkeit besteht in der mikrofluidisch unterstützten Kultivierung von Zellen in Monolayer- vorzugsweise aber in 3D-Kultur auf diversen Trägern. Dabei wird von 3D-Kulturen in mikrostrukturierten Trägern (3D-CellChips) ausgegangen, die sich bereits in der Erprobung auch für eine Langzeit-Kultivierung befinden. Als 3D-CellChips wird dabei eine flächige oder drei dimensional ausgeführte Unterstützungsstruktur zur dreidimensionalen Kultivierung von Zellen, die vorzugsweise perforiert ist und mit Medium durchströmt werden kann, bezeichnet.

Ein solcher 3D-CellChip (Träger) ist z.B. aus der WO 93/07258 bekannt, wobei sich die Größenordnungen für die Stützgerüste in der fortgeschrittenen Zellkultur an den physiologischen Größen orientieren. Die Höhe des Stützgerüstes für Zellen in einer dreidimensionalen Kultur, die von beiden Seiten versorgt wird, sollte 300 µm nicht übersteigen, wenn die Zellschicht nicht aktiv durchströmt wird. Der Prototyp des 3D-CellChips beherbergt auf einer Fläche von 1 cm² ca. 900 (30 x 30) Microcontainer mit den Dimensionen 300 x 300 x 300 µm (L x B x H) bei einer Wandstärke von 50 µm. Der Boden besitzt Poren, die einen ungehinderten Stoffaustausch schon bei Superfusion gewährleisten, aber auch das Durchströmen des Zellverbandes mit Medium erlauben (Perfusion).

Der Einsatz von Mikrosystemtechniken zur Herstellung von Bioreaktoren hat sich als großer Vorteil zur definierten Kultivierung von fortschrittlichen Zellkulturen herausgestellt. Die experimentell notwendige Diversität bezüglich verschiedener Zelllinien, Seren, Medien und Wirkstoffe kann ökonomisch nur durch die Anwendung miniaturisierter Systeme adressiert werden.

Aus der Dissertation von C. Augspurger (Leipzig 2007) ist bekannt, dass derartige 3D-CellChips (d.h. Probenträger mit 3D-Kulturen) standardmäßig in Bioreaktoren mit einigen 25 ml Volumen mit externen Pumpen als in sich geschlossene Systeme betrieben werden. Diese Bioreaktoren sind jedoch nur eingeschränkt für Anwendungen u.a. in der Pharmaforschung einsetzbar, da sie nicht kompatibel zu den Schnittstellen der automatisierten Labortechnik wie z.B. dem Mikrotiterplattenformat (oder auch "MTP footprint") sind. Außerdem ist es nicht möglich, zu diesen Bioreaktoren Testsubstanzen automatisch zuzuführen. Sie sind zudem durch eine mangelnde Parallelisierbarkeit charakterisiert. Ein weiteres Problem besteht darin, dass das eingesetzte notwendige biologische Material bzw. Volumen für viele Aufgaben einfach zu groß ist, um sinnvoll für parallele Ansätze eingesetzt zu werden.

Für die angestrebte Miniaturisierung und Parallelisierung auf einem MTP footprint ist das Format, Volumen bzw. die Konstruktion der aus dem Stand der Technik bekannten Bioreaktoren insbesondere für Screening Anwendungen zu groß und aufwendig. Aus Wintermantel: Dreidimensionale Zellkulturen; TUM Mitteilungen 4-2006 (Oktober 2006) ist das Einbringen von 3D strukturierten Polymeren oder Polymerschäumen in 24- oder 96-er MTP als Supportstrukturen für die 3D Kultivierung als passive Systeme bekannt. Im Gegensatz zu den bisher bekannten Bioreaktoren verfügen diese Systeme aber über keine aktive Perfusion, kein aktives Durchströmen des Gewebe- und Zellmaterials, stellen also ein unzureichendes Modell für die Nachbildung der Organe im lebenden Organismus dar.

In der US 2007/0128715 A1 ist ein Perfusionsgerät beschrieben, das einen Probenträger zur Aufnahme von Zellen besitzt. Außerdem sind Eingangskanäle vorhanden, über welche die Zellen mit einem Substart versorgt werden können. Eine externe Pumpe kann zu Verstärkung der Perfusion angeschlossen werden. Aufgrund der erforderlichen externen Einheiten ist eine parallel Anordnung zahlreicher Perfusionsgeräte in einer integrierten Einheit, insbesondere im Format einer Mikrotiter-Platte nicht möglich.

Die US 2004/0229349 A1 beschreibt u.a. ein Gerät für die mikrofluidische Behandlung und Detektion von Partikeln, wie beispielsweise Zellen. Die Durchflutung von mehreren Zellkammern wird z.B. von einer gemeinsamen peristaltischen Pumpeinheit hervorgerufen, welche jedoch nicht näher erläutert wird. Die Zellkammern stehen in fluidischem Kontakt, sodass die völlig unabhängige Kultivierung unterschiedlicher Zellkulturen in parallelen Zellkammern nicht möglich ist.

Aufgabe der vorliegenden Erfindung ist es deshalb, die aus dem Stand der Technik bekannten Nachteile zu überwinden und einen Mikrobioreaktor sowie ein miniaturisierbares, paralleles Bioreaktorsystem für die 3D-Zellkultivierung für die Laborautomatisierung bzw. das automatisierte High-Content-Screening (HCS), also der automatischen Bestimmung vieler biologischer und physikalischer Parameter, bzw. den sogenannten High-Throughput-Screening (HTS) bereitzustellen.

Erfindungsgemäß gelingt die Lösung dieser Aufgabe mit einem Mikrobioreaktor nach Patentanspruch 1 bzw. einer Bioreaktor-Mikrotiter-Platte nach Anspruch 13.

Ein erfindungsgemäßer Mikrobioreaktor besteht im Wesentlichen aus einem Probenträger zur Aufnahme von Zellen; einem Bypass mit einer Kapillaröffnung zur Zugabe von Testreagenz und/oder zum Be- bzw. Entlüften des Mikrobioreaktors; einem integrierten Pumpmodul zur Perfusion des Mikrobioreaktors mit Testreagenz und/oder Be- bzw. Entgasen des Mikrobioreaktors; einem transparenten Sichtfenster und Fluidkanälen, die einen Kreislauf bilden.

Eine erfindungsgemäße Bioreaktor-Mikrotiter-Platte besteht aus einer Vielzahl von derartigen Mikrobioreaktoren.

Vorteilhafte Ausgestaltungen der Erfindung sind in den Unteransprüchen angegeben.

Die Erfindung wird im Folgenden anhand von Zeichnungen näher erläutert. In den Zeichnungen zeigen:
- Fig. 1 -: einen prinzipiellen Aufbau eines erfindungsgemäßen Mikrobioreaktors;
- Fig. 2 -: einen prinzipiellen Aufbau einer Membranpumpe;
- Fig. 3 -: mehrere Ansichten einer Peristaltikpumpe zur Veranschaulichung der Funktionsweise in mehreren Schritten;
- Fig. 4 -: eine perspektivische Explosionsdarstellung des prinzipiellen Aufbaus einer bevorzugten erfindungsgemäßen Bioreaktor- bzw. CellChip-Mikrotiter-Platte.

Im Folgenden werden zwei mögliche Lösungsvarianten zur Konstruktion von Mikrobioreaktoren mit integrierten mikroperforierten Unterstützungsstrukturen unterschieden: die aktiven bzw. davon unterschieden die passiven Systeme. Als aktive Systeme werden solche Systeme bezeichnet, bei denen ein Fluid durch eine im System integrierte Pumpe durch das System gepumpt und somit eine CellChip-Struktur bzw. die darin kultivierte Probe durchströmt wird. Passive Systeme hingegen bezeichnen Systeme, bei denen CellChip-Strukturen bzw. Proben in die Mikrobioreaktoren oder Kultivierungsgefäße eingebracht werden und Strömungen - falls überhaupt - durch externe Pumpensysteme etc. realisiert werden.

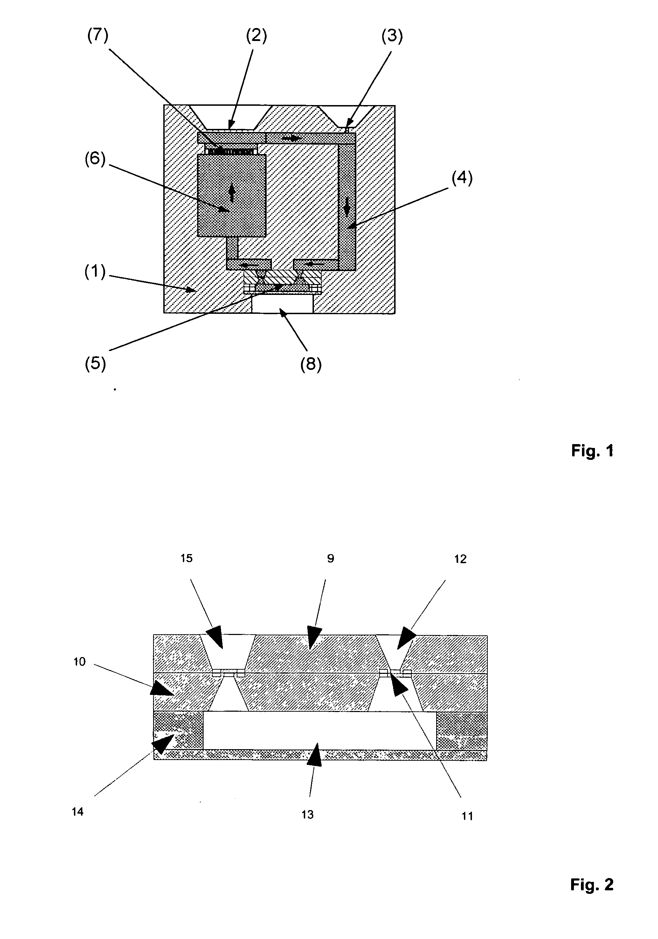

In Fig. 1 ist ein erfindungsgemäßer Mikrobioreaktor 1 in einer prinzipiellen Ausführungsform dargestellt. Der Mikrobioreaktor 1 besteht aus einem integrierten Pumpmodul 5, das z.B. als Membran- oder Peristaltikpumpe ausgeführt sein kann, einem Mediumreservoir 6 und einem Probenträger 7 (vorzugsweise ein 3D-CellChip) mit einer Probe, die von oben durch ein transparentes Fenster 2 beobachtet werden kann. Aufgrund des geringen Abstands zwischen dem Fenster 2 und der Probe ist ein Mikroskopieren mit geringem Objektivabstand möglich. Über einen Bypass 3 kann von außen in das System über einen Fluidkanal 4 Testreagens eingebracht werden. Der Fluidkanal 4 bildet über das Pumpmodul 5 und das Reservoir 6 einen Kreislauf aus, durch welchen das Fluid/Testreagenz gefördert wird, um die Probe am Probenträger 7 zu durchströmen. Über eine Zuleitung 8 kann ein in den Mikrobioreaktor integriertes Pumpmodul 5 betrieben werden, wobei eine pneumatische Aktivierung bzw. Ent- und Begasung möglich sind. Beim Betrieb des Pumpmoduls 5 fördert die Pumpe das Fluid im Kreislauf, sodass die Zellkultur (Probe) optimal mit dem Nährstoffmedium bzw. dem Testreagenz versorgt werden kann.

In einer abgewandelten Ausführungsform können vorzugsweise für Anwendungen in der Kokultur zwei CellChips übereinander gestapelt in den Bioreaktor eingebracht werden.

Der Probenträger 7 gestattet die zwei- oder vorzugsweise dreidimensionale Kultivierung von suspendierten oder aggregierten Einheiten, vorzugsweise Zellen oder Geweben. Der Probenträger kann dazu als Rückhaltemembran für suspendierte Zellen oder als Zellkultursubstrat für adhärente Zellen gestaltet sein. Für adhärente Zellen wird der Probenträger vorzugsweise auf ganzer Fläche gefüllt und nicht partiell, weil letzteres vorrangig eine Umströmung aber weniger die gewünschte Durchströmung der gefüllten Fläche zur Folge hätte.

In einer in Fig. 2 vereinfacht dargestellten Ausführungsform des erfindungsgemäßen Mikrobioreaktors ist eine Membranpumpe als Pumpmodul 5 vorgesehen. Sie besteht aus einem Ventilblock aus zwei gleichartigen Einzelteilen 9 und 10, die je nach verwendetem Material auch untrennbar gefügt sein können, wobei für die Gewährleistung der Funktion der Rückschlagventile, jeweils an einem Ventilsitz 11 eine besondere Beschichtung aufgebracht sein kann, die ein Verbinden an dieser Stelle verhindert. Über einen Zulauf 12 wird das Fluid eingesaugt, wenn sich eine Pumpkammer 13 durch eine Deformation einer Arbeitsmembran eines Kammergehäuses 14 vergrößert. Beim anschließenden Verkleinern des Kammervolumens wird das Fluid an einem Ablauf 15 wieder herausbefördert. Im quasistatischen Betrieb hat diese Pumpe nur eine Förderrichtung, welche durch die Anordnung der Ventile vorgegeben ist. Das Pumpmodul 5 ist derart in den Mikrobioreaktor integriert, dass eine parallele bzw. matrixartige Anordnung zahlreicher Mikrobioreaktoren mit jeweils integriertem Pumpmodul möglich bleibt.

Bei einer geeigneten Wahl von Ventilklappen, Kammergröße, Membran und Fluid kann die zuvor erwähnte Pumpe in Abhängigkeit von der Ansteuerfrequenz vor- bzw. rückwärts pumpen. Ein weiterer Vorteil der Verwendung einer Membranpumpe liegt in der aktiven Begasung des Mediums, da das Membranmaterial so gewählt werden kann, dass es für Gase permeabel ist. Das Kammervolumen der Membranpumpe ist abhängig von der gewünschten Förderrate und ist bei erfindungsgemäßen Mikrobioreaktoren vorzugsweise kleiner als 2µl.

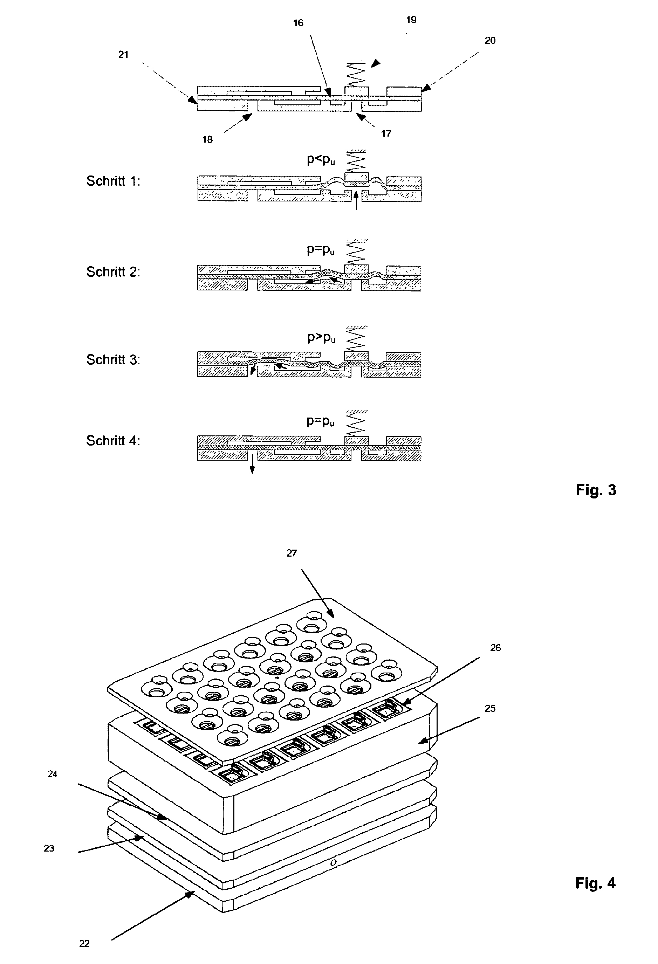

In einer weiteren Ausführungsform des erfindungsgemäßen Mikrobioreaktors ist eine Peristaltikpumpe als Pumpmodul 5 vorgesehen. Den Pumpzyklus der Peristaltikpumpe kann man grob in vier Abschnitte einteilen, die in Fig. 3 dargestellt sind. Zuerst wird über einer Arbeitsmembran 16 ein Unterdruck angelegt. Infolge dessen öffnet sich ein Zulauf 17 der Pumpe und der entstehende Unterdruck in der Pumpkammer wird durch das Einströmen von dem zu förderndem Fluid ausgeglichen. Wenn der anliegende Druck auf der Arbeitsmembran 16 den Umgebungsdruck erreicht hat, ist das Ventil am Zulauf 17 wieder geschlossen. Bei der weiteren Erhöhung des Druckes auf die Arbeitsmembran 16 strömt das zuvor angesaugte Medium in Richtung eines Ablaufs 18, da es auf Grund der Verdrängung für das Medium keine weitere Möglichkeit gibt. Im letzten Schritt liegt wieder der Ausgangszustand vor, d.h. am Ablauf 18 hat sich das Ventil aufgrund elastischer Effekte wieder geschlossen. Damit diese Art von Pumpe optimal arbeitet, wird sie in der Nähe der Resonanzfrequenz betrieben. Aufgrund der Phasenänderung beim Durchlaufen der Resonanzfrequenz kann auch die Pumprichtung umgekehrt werden.

Um die Begrenztheit der aus dem Stand der Technik bekannten Bioreaktoren und anderen Systeme zu überwinden, wird in einem parallelen Format erfindungsgemäß jedes Well, also jeder Mikrobioreaktor, mit einer Mikropumpe versehen, die konstruktionsgemäß als pulsierendes System die Situation in einem wirklichen Organismus (Herzschlag, Blutkreislauf) optimal nachbildet.

Ein solches System wird im Folgenden als "CellChip-Mikrotiter-Platte" (CellChip-MTP) oder Bioreaktor-Mikrotiter-Platte" (BR-MTP) bezeichnet. Eine CellChip-Mikrotiter-Platte besteht aus einem Array von vorzugsweise voneinander unabhängigen Mikrobioreaktoren, die als aktive Systeme ausgeführt sind. Kernstück einer solchen CellChip-Mikrotiter-Platte ist mindestens ein Mikrobioreaktor mit integrierter Pumpe vorzugsweise bestückt mit einem CellChip als Träger einer fortgeschrittenen Zellkultur. Dabei ist die Größe eines einzelnen Reaktors auf einer BR-MTP durch das Rastermaß einer Mikrotiter-Platte bestimmt in der je nach Ausführung 6, 12, 24, 48, 96 oder mehr Reaktoren parallel angeordnet sind. Das Medienvolumen, das sich innerhalb eines einzelnen Bioreaktors befindet, variiert somit je nach Menge der Reaktoren zwischen 100 µl und 25 ml, vorzugsweise aber im unteren Milliliter-Bereich. Alle Kanäle weisen vorzugsweise einen maximalen Querschnitt von unter 4mm² auf.

Ein bei allen geschlossenen Systemen ungelöstes Problem betrifft das automatische Einbringen von Testreagenz in das System. Gerade bei parallelisierten Anordnungen auf dem MTP footprint ist der Einsatz eines Pipettierroboters vorteilhaft, was in geschlossenen Systemen jedoch unzureichend möglich ist. In einem geschlossenen fluidischen System ist es normalerweise nicht möglich, über eine offene Kontaktstelle Flüssigkeit zuzudosieren. Das Fluidsystem ist über eine Kapillare, eine Art Bypass, mit der Umgebung verbunden. Diese Kapillare ermöglicht das Hinzufügen von kleinen Flüssigkeitsmengen. Erwartungsgemäß würde dies in einem vollständig gefluteten hydraulischen System nicht funktionieren. Da dieses System über elastische Elemente verfügt, kann es bis zu einem gewissen Grad Flüssigkeit aufnehmen. Die Menge der zugeführten Flüssigkeit hängt von der Größe der Kapillare und maximalen Deformation der elastischen Elemente ab, d.h. der Innendruck des Systems ist durch den Grenzflächendruck des Mediums zur Umgebung begrenzt. Elastische Elemente im Sinne der Erfindung können durch Fertigung des gesamten Systems aus einem elastischen Material (z.B. Silikon) erzeugt werden. Auch die Membran der Membranpumpe oder das gezielte Einbringen von Zonen mit sehr geringen Wandstärken kann in diesem Zusammenhang als elastisches Element betrachtet werden. Auch eine gezielte Oberflächenstrukturierung führt zu elastischem Verhalten des Systems.

Die Kapillare liegt in ihrer maximalen Dimension in der Größenordnung der Fluidkanäle. In einer teilsterilen und unsterilen Umgebung der BR-MTP kann ein Septum oder Sterilfilter zum Verschluss der Kapillare zum Einsatz kommen. Das Septum muss dabei in seiner Größe der Verwendung von marktüblichen Injektionskanülen genügen. Bei einer Abtrennung durch einen Sterilfilter sind dessen Dimensionen auch durch die Dimension der Fluidkanäle bestimmt. Das Filtermaterial muss gegenüber dem Testreagenz einen Kontaktwinkel von weniger als 90° und gegenüber dem Medium einen Kontaktwinkel von vorzugsweise größer 90° aufweisen. Bei einem Filter im Sinne der vorliegenden Erfindung muss es sich um eine Siebstruktur handeln, die das Eindringen kontaminierender Partikel verhindert (z.B. Bakterien, Zellen, Pilze, Staub,...). In einer sterilen Umgebung der BR-MTP kann die Kapillaröffnung auch unverschlossen sein. Hier ist die Dimension der Kapillaröffnung in der Größenordnung der Kanäle, vorzugsweise aber in einem Bereich <500µm, zu wählen. Die Kapillare muss so dimensioniert sein, dass der Innendruck des Systems (hervorgerufen durch die Pumpe) immer kleiner ist als der Krümmungsdruck des Meniskus, der an der Kapillare entsteht. Auch die Abdeckung der Kapillare mit einem einfachen Deckel ist Teil einer erfindungsgemäßen BR-MTP.

Erfindungsgemäß können basierend auf der Konstruktion hochintegrativer, paralleler Systeme mit aktiven Elementen zur Perfusion von 3D Zellkultursystemen in verschiedenen Ausführungsformen vorzugsweise mit integrierten Sensoren (pH, O₂, CO₂, ...) aktive Systeme auf HTS Anlagen, d.h. analog zu MTPs, realisiert werden.

Erste Versuchsanordnungen fokussieren auf ein 24well-Reaktorsystem. Ein solches aktives System ist potentiell transportfähig und wird gekapselt ausgeführt, also eine CellChip-MTP mit integrierter Fluidik mit zunächst 24 unabhängigen Bioreaktoren mit jeweils einer Öffnung und einem Bypass zur Zuführung z.B. von Testreagenzien und deren Verteilung im einzelnen Reaktor.

Dieses soll in einer Ausführung den Transport eukaryotischer Zellen vom Zellkulturlabor zum Anwender ermöglichen. Die Zellen werden in diesem Fall nicht tiefgefroren, sondern verbleiben in ihrer Kulturumgebung für eine optimale Kultur in einem Modul, das in eine Transportbox eingeschoben wird. Der Transport von einfachen Zellen ist sowohl in einfachen passiven als auch aktiven Systemen möglich. Hierzu werden im einfachsten Fall die 3D-CellChips (Proben) gehaust und mit der geeigneten Carbogen-Mischung (O₂ mit 5 % CO₂) begast und vorzugsweise auf einer einzustellenden gewünschten Temperatur gehalten.

In einer weiteren Ausführungsform kann das komplette Array in einem vereinfachten Fertigungsverfahren hergestellt und produziert werden. Eine solche Ausführungsform ist in Fig. 4 gezeigt. Diese Ausführungsform der erfindungsgemäßen Bioreaktor-Mikrotiter-Platte ist eine aktive 24 Wellplatte, bestehend aus einer Grundplatte 22 mit Anschluss für die Druckzufuhr. Eine Verteilerplatte 23 beinhaltet Kanäle für die Verteilung der Antriebsdruckluft auf die 24 Kanäle. Ein Pumpenhalter 24 nimmt die 24 Mikropumpen für die einzelnen Wells auf. Eine Rasterplatte 25 nimmt mehrere Träger 26 für die CellChips auf. Jeder Träger 26 stellt ein Kreislaufsystem des Mikrobioreaktors dar, welches der Aufnahme von Proben dient. Jedes Kreislaufsystem umfasst den Probenträger 7, die Fluidkanäle 4 und ggf. das Medienreservoir 6. Die Rasterplatte und die Kreislaufsysteme können auch zu einem Bauteil vereinigt werden. Das System wird mittels eines Deckels 27 abgeschlossen, in welchen die Sichtfenster 2 und die Zugänge zu den Bypässen 3 vorgesehen sind.

Der komplette CellChip-MTP kann demnach aus bis zu fünf einheitlichen Formkörpern produziert werden, wobei natürlich Zwischenstufen, bei denen z.B. die Membranen der einzelnen Pumpen oder die Pumpen als Einzelelemente in einen Rahmen gelegt werden, realisiert werden können.

In einer weiteren vorteilhaften Ausführungsform wird entweder durch chemische oder physikalische Oberflächenmodifikation der Fluidkanäle die Benetzung mit dem Fluid so optimiert, dass Gasblasen dort keine Anheftungsmöglichkeiten haben oder dass durch geometrische Minimierung relevanter Grenzflächen ebenso keine Gasblasen anheften können. Umgekehrt können in dem System gerade solche Stellen definiert werden, die als Blasenfänger dienen können.

Die Integration von Strömungssensoren ist in einer weiteren Ausführungsform realisierbar.

Deckgläschen zur Abdeckung des Bioreaktors können vorzugsweise als optisch transparente Sensoren, vorzugsweise aus dem AlGaN/GaN Material, ausgeführt sein. Das Sichtfenster besteht aus einem transparenten Sensor- und Funktionsmaterial, insbesondere aus Materialien der Gruppe III - V Halbleiter, wobei das transparente Sichtfenster (2) einer oder mehrere optisch transparente Sensoren umfasst.

### Bezugszeichenliste

| | |
|---|---|
| 1 | - Mikrobioreaktor |
| 2 | - transparentes Fenster |
| 3 | - Bypass mit Kapillaröffnung |
| 4 | - Fluidkanal |
| 5 | - Pumpmodul |
| 6 | - Mediumreservoir |
| 7 | - Probenträger |
| 8 | - Zuleitung |
| 9, 10 | - gleichartige Bauteile einer Membranpumpe |
| 11 | - Ventilsitz in der Membranpumpe |
| 12 | - Zulauf der Membranpumpe |
| 13 | - Pumpkammer der Membranpumpe |
| 14 | - Kammergehäuse mit Arbeitsmembran |
| 15 | - Ablauf der Membranpumpe |
| 16 | - Arbeitsmembran einer peristaltischen Pumpe |
| 17 | - Zulauf der peristaltischen Pumpe |
| 18 | - Ablauf der peristaltischen Pumpe |
| 19 | - Rückstellfeder |
| 20 | - Pumpendeckel |
| 21 | - Pumpenkörper |
| 22 | - Grundplatte mit Anschluss für eine Druckzufuhr |
| 23 | - Verteilerplatte |
| 24 | - Pumpenhalter |
| 25 | - Rasterplatte |
| 26 | - CellChip-Träger / Kreislaufsystem |
| 27 | - Deckel |

## Patentansprüche

1. Mikrobioreaktor (1) umfassend:
● einen Probenträger (7) zur Aufnahme einer Probe, insbesondere aus Zellen oder Geweben;
● ein integriertes Pumpmodul (5) zur Perfusion des Mikrobioreaktors mit einem Medium;
● Fluidkanäle (4), die zur Schaffung eines Kreislaufs für das Medium mindestens an das Pumpmodul (5) und den Probenträger (7) angeschlossen sind;
● einen Bypass (3) mit einer Kapillaröffnung, der zur Zugabe von Testreagenz und/oder zum Be- bzw. Entlüften des Mikrobioreaktors an den Kreislauf der Fluidkanäle (4) angeschlossen ist;
● ein transparentes Sichtfenster (2), welches die Beobachtung der Probe gestattet, wobei das Sichtfenster aus einem transparenten Sensor- und Funktionsmaterial, insbesondere aus einem Material der Gruppe III bis V Halbleiter, besteht, und wobei das transparente Sichtfenster (2) einen oder mehrere optisch transparente Sensoren umfasst.

2. Mikrobioreaktor nach Anspruch 1, **dadurch gekennzeichnet, dass** er weiterhin ein Mediumreservoir (6) besitzt, welches in den Kreislauf eingebunden ist.

3. Mikrobioreaktor nach Anspruch 1 oder 2, **dadurch gekennzeichnet, dass** das transparente Sichtfenster (2) und der Bypass (3) an einer Oberseite des Mikrobioreaktors angeordnet sind.

4. Mikrobioreaktor nach einem der Ansprüche 1 bis 3, **dadurch gekennzeichnet, dass** der Probenträger ein 3D-CellChip ist oder aus mehreren übereinander gestapelten 3D-CellChips besteht.

5. Mikrobioreaktor nach einem der Ansprüche 1 bis 4, **dadurch gekennzeichnet, dass** das Pumpmodul (5) eine peristaltische Pumpe mit einem Zu- bzw. Ablauf (17, 18) und einer Arbeitsmembran (16) ist, mit welcher eine Pumprichtungsumkehr realisiert werden kann.

6. Mikrobioreaktor nach einem der Ansprüche 1 bis 4, **dadurch gekennzeichnet, dass** das Pumpmodul (5) eine Membranpumpe mit zwei Rückschlagventilen (11) und einem Zu- bzw. Ablauf (12, 15) ist.

7. Mikrobioreaktor nach einem der Ansprüche 1 bis 6, **dadurch gekennzeichnet, dass** das Pumpmodul (5) eine permeable Arbeitsmembran (16) besitzt, durch welche ein Be- bzw. Entgasen des Mikrobioreaktors möglich ist.

8. Mikrobioreaktor nach einem der Ansprüche 1 bis 7, **dadurch gekennzeichnet, dass** das Pumpmodul (5) eine Zuleitung (8), insbesondere an einer Unterseite des Mikrobioreaktors besitzt, über welche ein Betriebsmittel zur Bestätigung des Pumpmodul zugeführt werden kann.

9. Mikrobioreaktor nach einem der Ansprüche 1 bis 8, **dadurch gekennzeichnet, dass** die Oberflächen der Fluidkanäle (4) grenxflachenminimierend ausgeführt sind.

10. Mikrobioreaktor nach einem der Ansprüche 1 bis 9, **dadurch gekennzeichnet, dass** sich unter- und/oder oberhalb des Probenträgers (7) eine Luftblasenfalle befindet, welche über einen direkt zugeführten Kanal befällt oder entleert werden kann.

11. Mikrobioreaktor nach Anspruch 10, **dadurch gekennzeichnet, dass** der zur Luftblasenfalle gerührte Kanal einen Verschluss besitzt, welcher insbesondere als ein Septum ausgebildet ist.

12. Mikrobioreaktor nach einem der Ansprüche 1 bis 11, **dadurch gekennzeichnet, dass** die optisch transparenten Sensoren, AlGaN-Sensoren sind.

13. Bioreaktor-Mikrotiter-Platte bestehend aus einer Vielzahl von matrixförmig angeordneten Mikrobioreaktoren (1) gemäß einem der Ansprüche 1 bis 12, deren durch ihre jeweiligen Fluidkanäle (4) gebildeten Kreisläufe unabhängig voneinander sind.

14. Bioreaktor-Mikrotiter-Platte nach Anspruch 13, **dadurch gekennzeichnet, dass** die Pumpmodule (5) aller Mikroreaktoren (1) jeweils eine eigene Zuleitung (8) für die Zufuhr eines Betriebsmediums besitzen.

15. Bioreaktor-Mikxotiter-Platte nach Anspruch 13 oder 14, **dadurch gekennzeichnet, dass** sie aus einem System von Formteilen, welche zu einer Einheit zusammengesetzt werden, besteht, wobei die einzelnen Formteile eine Grundplatte (22) mit Anschluss für eine Antriebsduckluftzufuhr, eine verteilerplatte (23) mit Kanälen für die Verteilung der Antriebsdruckluft, ein Pumpenhalter (24) zur Aufnahme der einzelnen Pumpmodule (5), eine Rasterplatte (25) zur Aufnahme einzelner Träger (26) und ein Deckel (27) sind, wobei die Bestandteile jedes einzelnen Mikroreaktors (1) auf den Pumpenhalte (24), die Träger (26) in der Rasterplatte (25) und den Deckel (27) verteilt sind.

## Claims

1. A microbioreactor (1) comprising:
● a sample support (7) for receiving a sample, particularly one made of cells or tissues;
● an integrated pump module (5) for the perfusion of the microbioreactor with a medium;
● fluid channels (4), which are connected for the establishment of a circuit for the medium at least to the pump module (5) and the sample support (7);
● a bypass (3) with a capillary opening which is connected for the addition of test reagent and/or for the ventilation or purging of the microbioreactor to the circuit of the fluid channels (4);
● a transparent viewing window (2) which allows the observation of the sample, whereas the viewing window consists of a transparent sensor material and functional material, particularly of a material of group III to V semiconductors, and whereas the transparent viewing window (2) comprises one or more optically transparent sensors.

2. A microbioreactor according to Claim 1, **characterized in that** it has moreover a media reservoir (6) which is incorporated in the circuit.

3. A microbioreactor according to Claim 1 or 2, **characterized in that** the transparent viewing window (2) and the bypass (3) are arranged on a top side of the microbioreactor.

4. A microbioreactor according to one of Claims 1-3, **characterized in that** the sample support is a 3D-CellChip or consists of several 3D-CellChips stacked one on top of the other.

5. A microbioreactor according to one of Claims 1-4, **characterized in that** the pump module (5) is a peristaltic pump with an inlet or outlet (17, 18) and a work membrane (16) by means of which a pump direction reversal can be achieved.

6. A microbioreactor according to one of Claims 1-4, **characterized in that** the pump module (5) is a membrane pump with two non-return valves (11) and an inlet or outlet (12, 15).

7. A microbioreactor according to one of Claims 1-6, **characterized in that** the pump module (5) has a permeable work membrane (16) through which gas feeding or purging of the microbioreactor is possible.

8. A microbioreactor according to one of Claims 1-7, **characterized in that** the pump module (5) has a feed line (8) particularly at a bottom side of the microbioreactor, through which an operating means can be supplied for the actuation of the pump module.

9. A microbioreactor according to one of Claims 1-8, **characterized in that** the surfaces of the fluid channels (4) are designed so as to minimize the boundary surface area.

10. A microbioreactor according to one of Claims 1-9, **characterized in that** an air bubble trap is located under and/or over the sample support (7), which trap can be filled or emptied through a channel leading directly into it.

11. A microbioreactor according to Claim 10, **characterized in that** the channel leading to the air bubble trap has a closure which is designed particularly as a septum.

12. A microbioreactor according to one of Claims 1-11, **characterized in that** the optically transparent sensors are AlGaN sensors.

13. A bioreactor microtiter plate consisting of a plurality of microbioreactors (1) according to one of Claims 1-12 arranged in a matrix pattern, whose circuits, which are formed in each case by their fluid channels (4), are independent of each other.

14. A bioreactor microtiter plate according to Claim 13, **characterized in that** the pump modules (5) of all the microbioreactors (1) each have their own feed line (8) for supplying an operating medium.

15. A bioreactor microtiter plate according to one of Claims 13 or 14, **characterized in that** it consists of a system of formed parts that are assembled to a unit, where the individual formed parts are a base plate (22) with connection for a driving pressurized air feed, a distribution plate (23) with channels for the distribution of the driving pressurized air, a pump holder (24) for the reception of the individual pump modules (5), a grid plate (25) for the reception of individual supports (26), and a cover (27), where the components of each individual microbioreactor (1) are distributed on the pump holder (24), the support (26) in the grid plate (25), and the cover (27).

## Revendications

1. Réacteur microbiologique (1) comprenant :
● un porte-échantillon (7) destiné à recevoir un échantillon, notamment constitué de cellules ou de tissus ;
● un module de pompage (5) intégré pour la perfusion du réacteur microbiologique avec un milieu ;
● des canaux à fluide (4), qui pour créer un circuit pour le milieu sont raccordés au moins au module de pompage (5) et au porte-échantillon (7) ;
● un conduit de dérivation (3) avec un orifice capillaire, qui pour l'addition d'un réactif de test et/ou pour l'aération ou la désaération du réacteur microbiologique est raccordé sur le circuit des canaux à fluide (4) ;
● un regard (2) transparent, qui permet l'observation de l'échantillon, le regard étant constitué d'une matière de capteur ou matière fonctionnelle transparente, notamment d'une matière en semiconducteurs des groupes III à V et le regard (2) transparent comprenant un ou plusieurs capteurs optiquement transparents.

2. Réacteur microbiologique selon la revendication 1, **caractérisé en ce qu'**il comporte en outre un réservoir à milieu (6) lequel est intégré dans le circuit.

3. Réacteur microbiologique selon la revendication 1 ou la revendication 2, **caractérisé en ce que** le regard (2) transparent et le conduit de dérivation (3) sont disposés sur une face supérieure du réacteur microbiologique.

4. Réacteur microbiologique selon l'une quelconque des revendications 1 à 3, **caractérisé en ce que** le porte-échantillon est une puce cellulaire 3D ou est constitué de plusieurs puces cellulaires 3 D empilées les unes au-dessus des autres.

5. Réacteur microbiologique selon l'une quelconque des revendications 1 à 4, **caractérisé en ce que** le module de pompage (5) est une pompe péristaltique avec une alimentation ou évacuation (17, 18) et une membrane de travail (16) permettant de réaliser une inversion de la direction de pompage.

6. Réacteur microbiologique selon l'une quelconque des revendications 1 à 4, **caractérisé en ce que** le module de pompage (5) est une pompe à membrane avec deux clapets antiretour (11) et une alimentation ou évacuation (12, 15).

7. Réacteur microbiologique selon l'une quelconque des revendications 1 à 6, **caractérisé en ce que** le module de pompage (5) comporte une membrane de travail (16) perméable à travers laquelle un gazage ou un dégazage du réacteur microbiologique est possible.

8. Réacteur microbiologique selon l'une quelconque des revendications 1 à 7, **caractérisé en ce que** le module de pompage (5) est muni d'un conduit d'alimentation (8), notamment sur une face inférieure du réacteur microbiologique, par l'intermédiaire duquel un consommable pour l'actionnement du module de pompage peut être alimenté.

9. Réacteur microbiologique selon l'une quelconque des revendications 1 à 8, **caractérisé en ce que** les surfaces des canaux à fluide (4) sont conçues de manière à réduire les surfaces limites.

10. Réacteur microbiologique selon l'une quelconque des revendications 1 à 9, **caractérisé en ce qu'**il se forme en-dessous ou au-dessus du porte-échantillon (7) un piège à bulle d'air qui peut être rempli ou vidé par l'intermédiaire d'un canal à amenage direct.

11. Réacteur microbiologique selon la revendication 10, **caractérisé en ce que** le canal tiré vers le piège à bulle d'air est muni d'une fermeture, laquelle est conçue notamment en tant que septum.

12. Réacteur microbiologique selon l'une quelconque des revendications 1 à 11, **caractérisé en ce que** les capteurs optiquement transparents sont des capteurs AlGaN.

13. Plaque de microtitrage pour réacteur biologique constituée d'une pluralité de réacteurs microbiologiques (1) disposés en forme de matrice selon l'une quelconque des revendications 1 à 12, dont les circuits formés par leurs canaux à fluides (4) respectifs sont indépendants les uns des autres.

14. Plaque de microtitrage pour réacteur biologique selon la revendication 13, **caractérisée en ce que** les modules de pompage (5) de tous les réacteurs microbiologiques (1) sont munis chacun d'un propre conduit d'alimentation (8) pour l'amenage d'un milieu d'exploitation.

15. Plaque de microtitrage pour réacteur biologique selon la revendication 13 ou la revendication 14, **caractérisée en ce qu'**elle est constituée d'un système de pièces moulées que l'on assemble en une unité, chaque pièce moulée étant une plaque de base (22) avec un raccord pour une alimentation d'un air comprimé d'entraînement, une plaque distributrice (23) avec des canaux pour la distribution de l'air comprimé d'entraînement, un support de pompe (24) destiné à recevoir les modules de pompage individuels (5), une plaque à crans (25) destinée à recevoir des supports (26) individuels et un couvercle (27), les composants de chaque réacteur microbiologique (1) étant distribués sur le support de pompe (24), les supports (26) dans la plaque à crans (25) et le couvercle (27).
